(19) Europäisches Patentamt
European Patent Office
Office européen des brevets

(11) EP 1 561 751 A1

(12) **EUROPÄISCHE PATENTANMELDUNG**

(43) Veröffentlichungstag:
**10.08.2005 Patentblatt 2005/32**

(21) Anmeldenummer: **05004525.1**

(22) Anmeldetag: **21.01.2003**

(51) Int Cl.[7]: **C07D 451/12**, A61K 31/439, A61P 1/06, A61P 11/06, A61P 13/06

(84) Benannte Vertragsstaaten:
**AT BE BG CH CY CZ DE DK EE ES FI FR GB GR HU IE IT LI LU MC NL PT SE SI SK TR**
Benannte Erstreckungsstaaten:
**AL LT LV MK RO**

(30) Priorität: **31.01.2002 DE 10203741**

(62) Dokumentnummer(n) der früheren Anmeldung(en) nach Art. 76 EPÜ:
**03734600.4 / 1 472 251**

(71) Anmelder: **Boehringer Ingelheim Pharma GmbH & Co.KG**
**55216 Ingelheim am Rhein (DE)**

(72) Erfinder:
- **Germeyer, Sabine**
  **Sandy Hook CT 06482 (US)**
- **Meissner, Helmut**
  **55218 Ingelheim (DE)**
- **Morschhaeuser, Gerd**
  **88400 Biberach (DE)**
- **Pestel, Sabine**
  **88448 Attenweiler (DE)**
- **Pieper, Michael, P.**
  **88400 Biberach (DE)**
- **Pohl, Gerald**
  **88400 Biberach (DE)**
- **Reichl, Richard**
  **55435 Gau-Algesheim (DE)**
- **Speck, Georg**
  **55218 Ingelheim (DE)**

Bemerkungen:
Diese Anmeldung ist am 02 - 03 - 2005 als Teilanmeldung zu der unter INID-Kode 62 erwähnten Anmeldung eingereicht worden.

(54) **Fluorencarbonsäureester, Verfahren zu deren Herstellung sowie deren Verwendung als Arzneimittel**

(57) Die vorliegende Erfindung betrifft neue Fluorencarbonsäureester der allgemeinen Formel 1

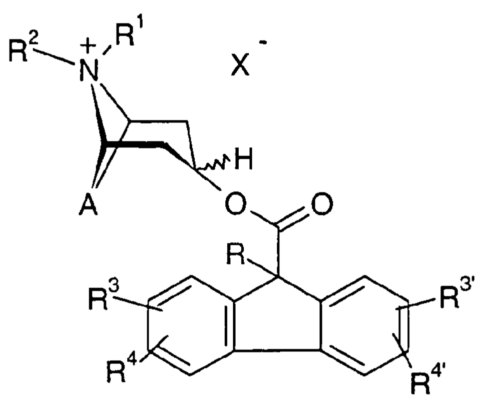

**1**

worin X - und die Gruppen A, R, $R^1$, $R^2$, $R^3$, $R^{3'}$, $R^4$ und $R^{4'}$ die in den Ansprüchen und in der Beschreibung genannten Bedeutungen haben können, Verfahren zu deren Herstellung sowie deren Verwendung als Arzneimittel.

EP 1 561 751 A1

**Beschreibung**

Die vorliegende Erfindung betrifft neue Fluorencarbonsäureester der allgemeinen Formel 1

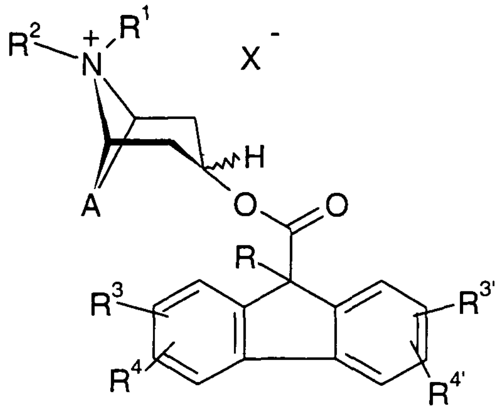

1

worin $X^-$ und die Gruppen A, R, $R^1$, $R^2$, $R^3$, $R^{3'}$, $R^4$ und $R^{4'}$ die in den Ansprüchen und in der Beschreibung genannten Bedeutungen haben können, Verfahren zu deren Herstellung sowie deren Verwendung als Arzneimittel.

Beschreibung der Erfindung

Die vorliegende Erfindung betrifft Verbindungen der allgemeinen Formel 1

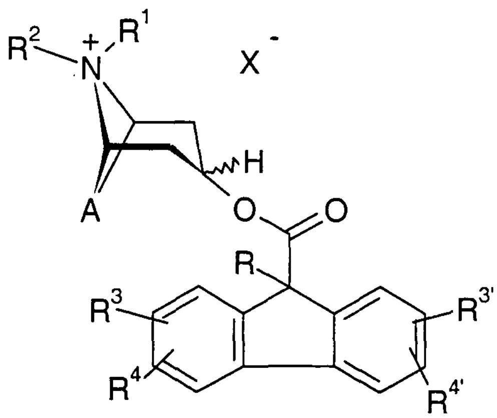

1

worin

A ein zweibindiger Rest ausgewählt aus der Gruppe bestehend aus

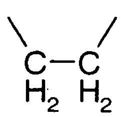 , 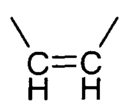 und 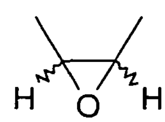 ;

| | |
|---|---|
| $X^-$ | ein einfach negativ geladenes Anion, vorzugsweise ein Anion ausgewählt aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat; |
| R | Wasserstoff, Hydroxy, Methyl, Ethyl, $-CF_3$, $CHF_2$ oder Fluor; |
| $R^1$ und $R^2$ | gleich oder verschieden, $-C_1$-$C_5$-Alkyl, welches gegebenenfalls durch $-C_3$-$C_6$-Cycloalkyl, Hydroxy oder Halogen substituiert sein kann, oder |

$R^1$ und $R^2$ gemeinsam eine -$C_3$-$C_5$-Alkylen-Brücke;

$R^3$, $R^4$, $R^{3'}$ und $R^{4'}$, gleich oder verschieden, Wasserstoff, -$C_1$-$C_4$-Alkyl, -$C_1$-$C_4$-Alkyloxy, Hydroxy, -$CF_3$, -$CHF_2$, CN, $NO_2$ oder Halogen bedeuten.

Bevorzugt sind Verbindungen der allqemeinen Formel 1, worin

A ein zweibindiger Rest ausgewählt aus der Gruppe bestehend aus

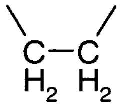 , 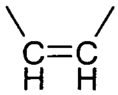 und 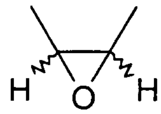 ;

X⁻ ein einfach negativ geladenes Anion ausgewählt aus der Gruppe Chlorid, Bromid, 4-Toluolsulfonat und Methansulfonat, bevorzugt Bromid;

R Hydroxy, Methyl oder Fluor;

$R^1$ und $R^2$ gleich oder verschieden, Methyl, Ethyl oder Fluorethyl;

$R^3$, $R^4$, $R^{3'}$ und $R^{4'}$, gleich oder verschieden, Wasserstoff, Methyl, Methyloxy, Hydroxy, -$CF_3$, -$CHF_2$ oder Fluor bedeuten.

Besonders bevorzugt sind Verbindungen der allgemeinen Formel <u>**1**</u>, worin

A ein zweibindiger Rest ausgewählt aus der Gruppe bestehend aus

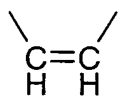 und 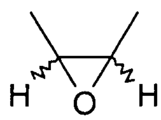 ;

X⁻ ein einfach negativ geladenes Anion ausgewählt aus der Gruppe Chlorid, Bromid und Methansulfonat, bevorzugt Bromid;

R Hydroxy, Methyl oder Fluor, bevorzugt Methyl oder Hydroxy;

$R^1$ und $R^2$ gleich oder verschieden, Methyl oder Ethyl, bevorzugt Methyl;

$R^3$, $R^4$, $R^{3'}$ und $R^{4'}$, gleich oder verschieden, Wasserstoff, -$CF_3$, -$CHF_2$ oder Fluor, bevorzugt Wasserstoff oder Fluor bedeuten.

Erfindungsgemäß von besonderer Bedeutung sind Verbindungen der allgemeinen Formel <u>**1**</u>, worin

A ein zweibindiger Rest ausgewählt aus der Gruppe bestehend aus

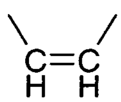 und 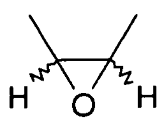 ;

X⁻ Bromid;

R Hydroxy oder Methyl, bevorzugt Methyl;

$R^1$ und $R^2$ gleich oder verschieden, Methyl oder Ethyl, bevorzugt Methyl;

$R^3$, $R^4$, $R^{3'}$ und $R^{4'}$, gleich oder verschieden, Wasserstoff, oder Fluor bedeuten. Gegenstand der Erfindung sind die jeweiligen Verbindungen der Formel <u>**1**</u> gegebenenfalls in Form der einzelnen optischen Isomeren, Mischungen der einzelnen Enantiomeren oder Racemate.

In den Verbindungen der allgemeinen Formel <u>**1**</u> können die Reste $R^3$, $R^4$, $R^{3'}$ und $R^{4'}$, sofern sie nicht Wasserstoff bedeuten, jeweils ortho, meta oder para bezüglich der Verknüpfung zur "-C- R"-Gruppe angeordnet sein. Sofern keiner der Reste $R^3$, $R^4$, $R^{3'}$ und $R^{4'}$ Wasserstoff bedeutet, sind $R^3$und $R^{3'}$ bevorzugt in para-Position und $R^4$ und $R^{4'}$ bevorzugt in ortho- oder meta-Position, besonders bevorzugt in meta-Position verknüpft. Sofern einer der Reste $R^3$ und $R^4$ und einer der Reste $R^{3'}$ und $R^{4'}$ Wasserstoff bedeutet, ist der jeweils andere Rest bevorzugt in meta- oder para-Position, besonders bevorzugt in para-Position verknüpft. Sofern keiner der Reste $R^3$, $R^4$, $R^{3'}$ und $R^{4'}$ Wasserstoff bedeutet sind erfindungsgemäß die Verbindungen der allgemeinen Formel 1 besonders bevorzugt, in denen die Reste $R^3$, $R^4$, $R^{3'}$ und $R^{4'}$ dieselbe Bedeutung aufweisen.

Von besonderer Bedeutung sind erfindungsgemäß ferner diejenigen Verbindungen der allgemeinen Formel 1, in denen der Ester-substituent am Stickstoff-bicylus α-konfiguriert ist. Diese Verbindungen entsprechen der allgemeinen Formel **1-α**

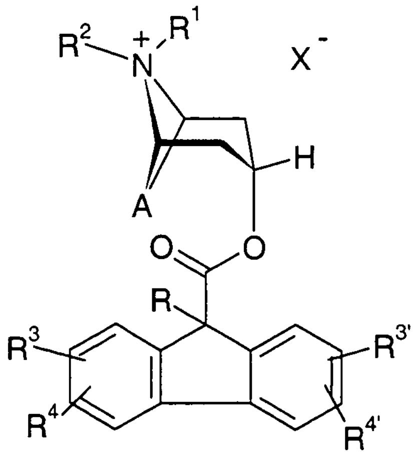

**1-α**.

Die nachfolgenden Verbindungen sind erfindungsgemäß von besonderer Bedeutung:

- 9-Hydroxy-fluoren-9-carbonsäuretropenolester -Methobromid ;
- 9-Fluor-fluoren-9-carbonsäuretropenolester -Methobromid ;
- 9-Hydroxy-fluoren-9-carbonsäurescopinester -Methobromid ;
- 9-Fluor-fluoren-9-carbonsäurescopinester Methobromid ;
- 9-Methyl-fluoren-9-carbonsäuretropenolester Methobromid ;
- 9-Methyl-fluoren-9-carbonsäurescopinester Methobromid ;

Als Alkylgruppen werden, soweit nicht anders angegeben, verzweigte und unverzweigte Alkylgruppen mit 1 bis 5 Kohlenstoffatomen bezeichnet. Beispielsweise werden genannt: Methyl, Ethyl, Propyl oder Butyl. Zur Bezeichnung der Gruppen Methyl, Ethyl, Propyl oder auch Butyl werden gegebenenfalls auch die Abkürzungen Me, Et, Prop oder Bu verwendet. Sofern nicht anders beschrieben, umfassen die Definitionen Propyl und Butyl alle denkbaren isomeren Formen der jeweiligen Reste. So umfaßt beispielsweise Propyl n-Propyl und iso-Propyl, Butyl umfaßt iso-Butyl, sec. Butyl und tert.-Butyl etc.

Als Alkylengruppen werden, soweit nicht anders angegeben, verzweigte und unverzweigte zweibindige Alkylbrücken mit 1 bis 4 Kohlenstoffatomen bezeichnet. Beispielsweise werden genannt: Methylen, Ethylen, Propylen oder Butylen.

Als Alkylen-Halogen-Gruppen werden, soweit nicht anders angegeben, verzweigte und unverzweigte zweibindige Alkylbrücken mit 1 bis 4 Kohlenstoffatomen bezeichnet, die ein-, zwei- oder dreifach, bevorzugt zweifach durch ein Halogen substituiert sind. Dementsprechend werden als Alkylen-OH-Gruppen, soweit nicht anders angegeben, verzweigte und unverzweigte zweibindige Alkylbrücken mit 1 bis 4 Kohlenstoffatomen bezeichnet, die ein-, zwei- oder dreifach, bevorzugt einfach durch ein Hydroxy substituiert sind.

Als Alkyloxygruppen werden, soweit nicht anders angegeben, verzweigte und unverzweigte Alkylgruppen mit 1 bis 4 Kohlenstoffatomen bezeichnet, die über ein Sauerstoffatom verknüpft sind. Beispielsweise werden genannt: Methylox, Ethyloxy, Propyloxy oder Butyloxy. Zur Bezeichnung der Gruppen Methyloxy, Ethyloxy, Propyloxy oder auch Butyloxy werden gegebenenfalls auch die Abkürzungen MeO-, EtO-, PropO- oder BuO- verwendet. Sofern nicht anders beschrieben, umfassen die Definitionen Propyloxy und Butyloxy alle denkbaren isomeren Formen der jeweiligen Reste. So umfaßt beispielsweise Propyloxy n-Propyloxy und iso-Propyloxy, Butyloxy umfaßt iso-Butyloxy, sec. Butyloxy und tert.-Butyloxy etc. Gegebenenfalls wird im Rahmen der vorliegenden Erfindung statt der Bezeichnung Alkyloxy auch die Bezeichnung Alkoxy verwendet. Zur Bezeichnung der Gruppen Methyloxy, Ethyloxy, Propyloxy oder auch Butyloxy gelangen dementsprechend gegebenenfalls auch die Ausdrücke Methoxy, Ethoxy, Propoxy oder Butoxy zur Anwendung.

Als Alkylen-alkyloxy-Gruppen werden, soweit nicht anders angegeben, verzweigte und unverzweigte zweibindige Alkylbrücken mit 1 bis 4 Kohlenstoffatomen bezeichnet, die ein-, zwei- oder dreifach, bevorzugt einfach durch eine Alkyloxygruppe substituiert sind.

Als -O-CO-Alkylgruppen werden, soweit nicht anders angegeben, verzweigte und unverzweigte Alkylgruppen mit 1 bis 4 Kohlenstoffatomen bezeichnet, die über eine Estergruppe verknüpft sind. Dabei sind die Alkylgruppen direkt an den Carbonylkohlenstoff der Estergruppe gebunden. In analoger Art und Weise ist die Bezeichnung -O-CO-Alkyl-Halogen-gruppe zu verstehen. Die Gruppe $-O-CO-CF_3$ steht für Trifluoracetat.

Halogen steht im Rahmen der vorliegenden Erfindung für Fluor, Chlor, Brom oder Jod. Sofern nicht gegenteilig angegeben, gelten Fluor und Brom als bevorzugte Halogene. Die Gruppe CO bezeichnet eine Carbonylgruppe.

Die Herstellung der erfindungsgemäßen Verbindungen kann, wie nachstehend erläutert, zum Teil in Analogie zu im Stand der Technik bereits bekannten Vorgehensweisen erfolgen (Schema 1). Die Carbonsäurederivate der Formel 3 sind im Stand der Technik bekannt oder können nach im Stand der Technik bekannten Syntheseverfahren erhalten werden. Sind lediglich geeignet substituierte Carbonsäuren im Stand der Technik bekannt, können die Verbindungen der Formel 3 auch direkt aus diesen durch Säure- oder Basen-katalysierte Veresterung mit den entsprechenden Alkoholen oder durch Halogenierung mit den entsprechenden Halogenierungsreagentien erhalten werden.

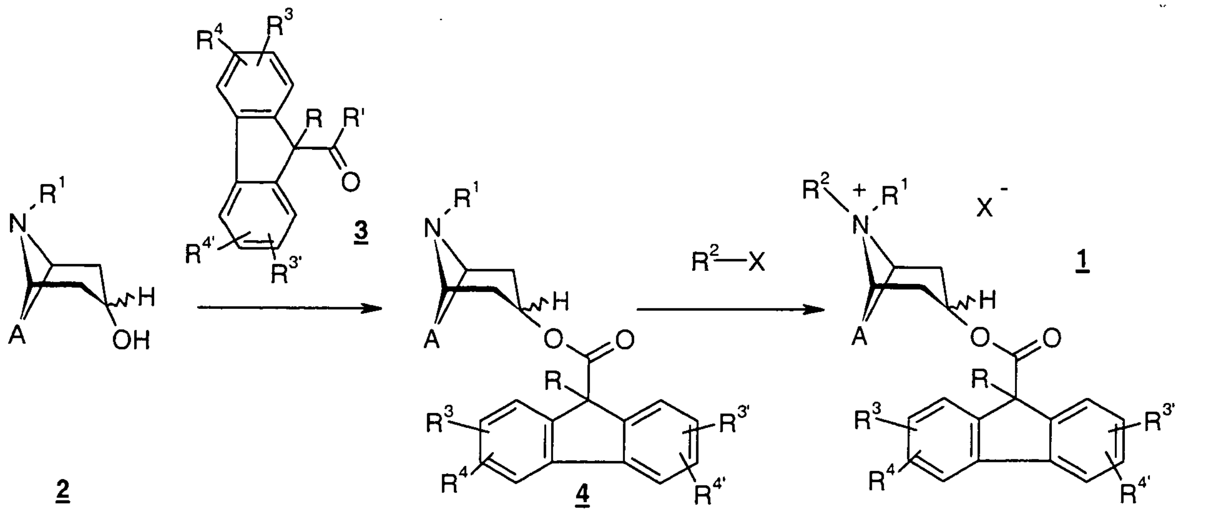


2 3 4 1

Schema 1:

Wie aus Schema 1 ersichtlich, dienen als Ausgangsprodukte für die Darstellung der Verbindungen der Formel 1 die Verbindungen der Formel 2. Diese Verbindungen sind im Stand der Technik bekannt.

Ausgehend von den Verbindungen der Formel 2 gelingt der Zugang zu den Estern der allgemeinen Formel 4 durch Umsetzung mit den Carbonsäurederivaten der Formel 3, in denen R' beispielsweise für Chlor oder einen $C_1$-$C_4$-Alkyloxyrest steht. Im Falle von R' gleich $C_1$-$C_4$-Alkyloxy kann diese Umsetzung beispielsweise in einer Natriumschmelze bei erhöhter Temperatur, bevorzugt bei ca. 50-150°C, besonders bevorzugt bei etwa 90-100°C bei niedrigem Druck, bevorzugt bei unter 500mbar, besonders bevorzugt bei unter 75mbar durchgeführt werden. Alternativ dazu können statt der Derivate 3, in denen R' $C_1$-$C_4$-Alkyloxy bedeutet auch die entsprechenden Säurechloride (R gleich Cl) eingesetzt werden.

Die so erhaltenen Verbindungen der Formel 4 lassen sich durch Umsetzung mit den Verbindungen $R^2$-X, in denen $R^2$ und X die vorstehend genannten Bedeutungen haben können, in die Zielverbindungen der Formel 1 überführen. Auch die Durchführung dieses Syntheseschritts kann in Analogie zu den in der WO 92/16528 offenbarten Synthesebeispielen erfolgen. In dem Fall in dem $R^1$ und $R^2$ gemeinsam eine Alkylenbrücke bilden, ist, wie für den Fachmann ersichtlich, die Zugabe des Reagenzes $R^2$-X nicht erforderlich. In diesem Fall weisen die Verbindungen der Formel 4 einen geeignet substituierten Rest $R^1$ (beispielsweise $-C_3$-$C_5$-Alkylen-Halogen) entsprechend der vorstehend genannten Definitionen auf und die Darstellung der Verbindungen der Formel 1 erfolgt durch intramolekulare Quarternierung des Amins.

Alternativ zu der in Schema 1 dargestellten Vorgehensweise zur Synthese der Verbindungen der Formel 4 lassen sich die Derivate 4, in denen der Stickstoffbicyclus ein Scopin-Derivat darstellt, durch Oxidation (Epoxidierung) von Verbindungen der Formel 4 erhalten, in denen der Stickstoffbicyclus ein Tropenyl-Rest ist. Hierzu kann erfindungsgemäß wie folgt vorgegangen werden.

Die Verbindung 4, in der A für -CH=CH- steht, wird in einem polaren organischen Lösemittel, bevorzugt in einem Lösemittel ausgewählt aus der Gruppe N-Methyl-2-pyrrolidon (NMP), Dimethylacetamid und Dimethylformamid, bevorzugt Dimethylformamid suspendiert und anschließend erwärmt auf eine Temperatur von ca. 30-90°C, vorzugsweise 40-70°C. Anschließend wird ein geeignetes Oxidationsmittel zugegeben und bei konstanter Temperatur 2 bis 8 Stunden, bevorzugt 3 bis 6 Stunden gerührt. Als Oxidationsmittel kommt bevorzugt Vanadiumpentoxid im Gemisch mit $H_2O_2$, besonders bevorzugt $H_2O_2$-Harnstoffkomplex in Kombination mit Vanadiumpentoxid zur Anwendung. Die Aufarbeitung erfolgt auf üblichem Wege. Die Reinigung der Produkte kann je nach Kristallisationsneigung durch Kristallisation oder Chromatographie erfolgen.

Alternativ dazu sind die Verbindungen der Formel 4, in denen R Halogen bedeutet auch auf dem in Schema 2 dargestellten Wege zugänglich.

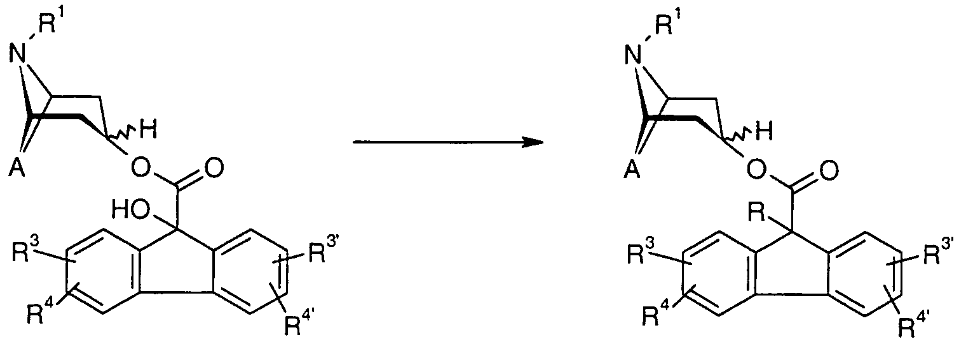

4 (mit R= OH) 4 (mit R= Halogen)

Schema 2:

Hierzu werden die Verbindungen der Formel 4 in denen R für Hydroxy steht unter Verwendung geeigneter Halogenierungsreagentien in die Verbindungen 4, in denen R Halogen bedeutet, überführt. Die Durchführung der nach Schema 2 durchzuführenden Halogenierungsreaktionen ist im Stand der Technik hinreichend bekannt.

Wie in Schema 1 ersichtlich, kommt den Zwischenprodukten der allgemeinen Formel 4 eine zentrale Bedeutung zu. Dementsprechend zielt ein weiterer Aspekt der vorliegenden Erfindung auf die Intermediate der Formel 4

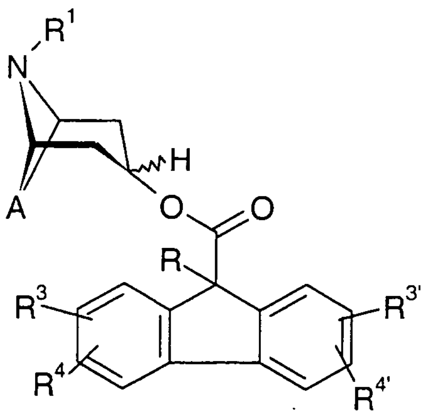

4

worin die Reste A, R, $R^1$, $R^3$, $R^{3'}$, $R^4$und $R^{4'}$ die vorstehend genannten Bedeutungen haben können, gegebenenfalls in Form ihrer Säureadditionssalze. Unter Säureadditionssalzen werden dabei Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-ptoluolsulfonat, bevorzugt Hydrochlorid, Hydrobromid, Hydrosulfat, Hydrophosphat, Hydrofumarat und Hydromethansulfonat verstanden.

Wie in den Verbindungen der allgemeinen Formel 1 können auch in den Verbindungen der allgemeinen Formel 4 die Reste $R^3$, $R^4$, $R^{3'}$ und $R^{4'}$, sofern sie nicht Wasserstoff bedeuten, jeweils ortho, meta oder para bezüglich der Verknüpfung zur "-C- R"-Gruppe angeordnet sein. Sofern keiner der Reste $R^3$, $R^4$, $R^{3'}$ und $R^{4'}$ Wasserstoff bedeutet, sind $R^3$und $R^{3'}$ bevorzugt in para-Position und $R^4$ und $R^{4'}$ bevorzugt in ortho- oder meta-Position, besonders bevorzugt

in meta-Position verknüpft. Sofern einer der Reste $R^3$ und $R^4$ und einer der Reste $R^{3'}$ und $R^{4'}$ Wasserstoff bedeutet, ist der jeweils andere Rest bevorzugt in meta- oder para-Position, besonders bevorzugt in para-Position verknüpft. Sofern keiner der Reste $R^3$, $R^4$, $R^{3'}$ und $R^{4'}$ Wasserstoff bedeutet sind erfindungsgemäß die Verbindungen der allgemeinen Formel **4** besonders bevorzugt, in denen die Reste $R^3$, $R^4$, $R^{3'}$ und $R^{4'}$ dieselbe Bedeutung aufweisen.

Erfindungsgemäß bevorzugt werden die Verbindungen der Formel **4** in α-konfigurierter Form als Ausgangsmaterialien eingesetzt. Diese α-konfigurierten Verbindungen sind erfindungsgemäß demzufolge von besonderer Bedeutung und entsprechen der allgemeinen Formel **4-α.**

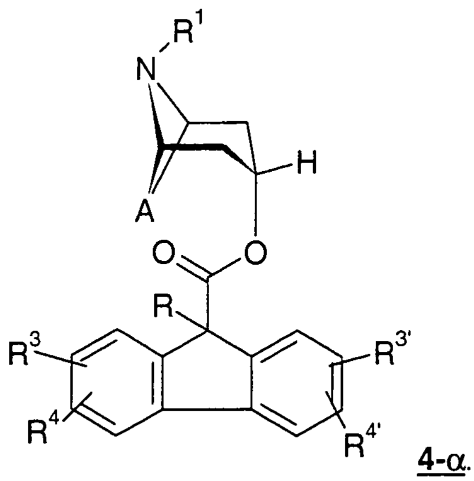

**4-α.**

Ein weiterer Aspekt der vorliegenden Erfindung betrifft die Verwendung von Verbindungen der allgemeinen Formel **2** zur Herstellung der Verbindungen der allgemeinen Formel **4**. Ferner betrifft die vorliegende Erfindung die Verwendung der Verbindungen der allgemeinen Formel **2** als Ausgangsmaterial zur Herstellung der Verbindungen der allgemeinen Formel **1**. Ferner betrifft die vorliegende Erfindung die Verwendung der Verbindungen der allgemeinen Formel **4** als Zwischenprodukt bei der Herstellung der Verbindungen der allgemeinen Formel **1.**

Die nachstehend beschriebenen Synthesebeispiele dienen der weitergehenden Illustration der vorliegenden Erfindung. Sie sind allerdings nur als exemplarische Vorgehensweisen zur weitergehenden Erläuterung der Erfindung zu verstehen, ohne selbige auf den nachfolgend exemplarisch beschriebenen Gegenstand zu beschränken.

**Beispiel 1:** 9-Hydroxy-fluoren-9-carbonsäuretropenolester -Methobromid :

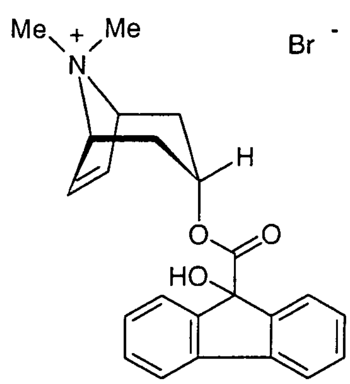

**1.1.:** 9-Hydroxy-fluoren-9-carbonsäuremethylester **3a:.**

50,4 g (0,223 mol) 9-Hydroxy-9-fluorencarbonsäure werden in 500 ml Methanol gelöst, mit 5 ml (0,089 mol) konz. Schwefelsäure versetzt und 1 Stunde unter Rückfluß erhitzt. Nach Abkühlen werden 100 ml Natriumhydrogencarbonatlösung (ca. pH 8) zugegeben und das Methanol weitgehend eingedampft. Es wird mit Dichlormethan und Wasser extrahiert, die organische Phase getrocknet und zur Trockene eingedampft. Die Reinigung erfolgt durch Umkristallisation aus Ethylacetat. Ausbeute: 50,0g weiße Kristalle (= 93% d. Th.).

**1.2:** 9-Hydroxy-fluoren-9-carbonsäuretropenolester **4a:**

13,4 g (0,056 mol) Methylester **3a,** 11,65 g (0,084 mol) Tropenol und 0,3 g Natrium werden als Schmelze bei 75 mbar 4 h auf kochendem Wasserbad unter gelegentlichem Schütteln erhitzt. Nach Abkühlung werden die Natriumreste mit Acetonitril aufgelöst, die Lösung zur Trockene eingedampft und der Rückstand mit Dichlormethan/Wasser extrahiert. Die organische Phase wird mit Wasser gewaschen, über $MgSO_4$ getrocknet und das Lösemittel abdestilliert. Die Reinigung des Produkts erfolgt durch Umkristallisation aus Diethylether.
Ausbeute: 11,40 g weiße Kristalle (= 59 % d. Th.).

**1.3:** 9-Hydroxy-fluoren-9-carbonsäuretropenolester -Methobromid :

1,75 g (0,005 mol) **4a** werden in 30 ml Dichlormethan und 15 ml Acetonitril aufgenommen und mit 2,85 g (0,015 mol) 50%-iges Methylbromidlösung in Acetonitril versetzt. Die Reaktionsmischung wird bei Raumtemperatur über 3 Tage stehen gelassen, wobei das Produkt kristallisiert. Die ausgefallenen Kristalle werden abgetrennt und zur Reinigung aus Diethylether umkristallisiert.
Ausbeute: 1,95 g weiße Kristalle (= 88 % d. Th); Schmp.: 250°C.

| | | | | |
|---|---|---|---|---|
| Elementaranalyse | berechnet | C (62,45) | H (5,47) | N (3,17) |
| | gefunden. | C (61,53) | H (5,84) | N (3,22). |

**Beispiel 2:** 9-Fluor-fluoren-9-carbonsäuretropenolester -Methobromid :

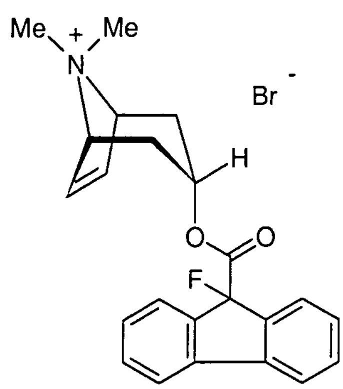

**2.1:** 9-Fluor-fluoren-9-carbonsäuretropenolester **4b:**

1,66 ml (0,009 mol) Bis-(2-methoxyethyl)-aminosulfurtrifluorid werden in 10 ml Dichlormethan vorgelegt und innerhalb von 20 Minuten bei 15°-20° C mit einer Lösung aus 2,4g (0,007 mol) **4a** in 25 ml Dichlormethan tropfenweise versetzt. Es wird 20 h bei Raumtemperatur gerührt, abgekühlt auf 0° C und vorsichtig mit 80 ml Wasser unter gutem Rühren versetzt. Anschließend wird vorsichtig mit wässeriger $NaHCO_3$-Lösung auf pH 8 eingestellt, die organische Phase abgetrennt, die Wasserphase erneut mit Dichlormethan extrahiert, die vereinten organischen Phasen mit Wasser gewaschen, über $MgSO_4$ getrocknet und zur Trockene eingedampft. Es wird das Hydrochlorid gefällt und aus Acetonitril/Diethylether umkristallisiert. Anschließend wird wieder die freie Base mittels 10% aq. Natriumcarbonatlsg. freigesetzt. Ausbeute: 1,05 g hellgelbe Kristalle (= 53 % d. Th.)

**2.2:** 9-Fluor-fluoren-9-carbonsäuretropenolester -Methobromid :

1,05 g (0,003 mol) **4b** werden in 20 ml Acetonitril aufgenommen und mit 1,71 g (0,009 mol) 50%-iges Methylbromidlösung in Acetonitril in Analogie zu Schritt 1.3 umgesetzt. Zur Reinigung wird aus Acetonitril umkristallisiert.
Ausbeute: 0,80 g weiße Kristalle (= 60 % d. Th); Schmp.: 252°C.

| | | | | |
|---|---|---|---|---|
| Elementaranalyse | berechnet | C (62,17) | H (5,22) | N (3,15) |
| | gefunden. | C (62,04) | H (5,23) | N (3,15). |

**Beispiel 3:** 9-Hydroxy-fluoren-9-carbonsäurescopinester -Methobromid :

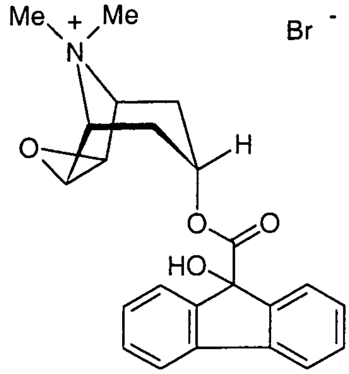

**3.1:** 9-Hydroxy-fluoren-9-carbonsäurescopinester **4c:**

9,0 g (0,026 mol) Tropenolester **4a** werden in 90 ml Dimethylformamid suspendiert und mit 0,47 g (0,003 mol) Vanadium-(V)-oxid versetzt. Bei 60°C wird eine Lösung aus 4,89 g (0,052 mol) $H_2O_2$-Harnstoff in 20 ml Wasser zugetropft und 6 Stunden bei 60°C gerührt. Nach Abkühlen auf 20°C wird der entstandene Niederschlag abgesaugt, das Filtrat mit 4 N Salzsäure auf pH 2 gestellt und mit $Na_2S_2O_5$ gelöst in Wasser versetzt. Die dadurch entstandene Lösung wird zur Trockene eingedampft, der Rückstand mit Dichlormethan/Wasser extrahiert. Die saure Wasserphase wird mit $Na_2CO_3$ basisch gestellt, mit Dichlormethan extrahiert und die organische Phase über $Na_2SO_4$ getrocknet und konzentriert.
Anschließend erfolgte die Zugabe von 1 ml Acetylchlorid bei Raumtemperatur und es wird 1 Stunde gerührt. Nach Extraktion mit 1 N Salzsäure wird die Wasserphase basisch gestellt, mit Dichlormethan extahiert, die organische Phase mit Wasser gewaschen und über $MgSO_4$ getrocknet. Das Lösemittel wird abschließend destillativ entfernt. Die Reinigung des Rohprodukts erfolgt durch Umkristallisieren aus Diethylether. Ausbeute: 2,8 g weiße Kristalle (= 30 % d. Th.).

**3.2:** 9-Hydroxy-fluoren-9-carbonsäurescopinester -Methobromid :

1,3 g (0,004 mol) **4c** werden in 20 ml Chloroform und 20 ml Acetonitril aufgenommen und mit 2,279 g (0,012 mol) 50%-iges Methylbromidlösung in Acetonitril in Analogie zu Schritt 1.3 umgesetzt. Zur Reinigung wird aus Acetonitril umkristallisiert. Ausbeute: 1,25 g hellbeige Kristalle (= 68 % d. Th); Schmp.: 243-244°C.

| | | | | |
|---|---|---|---|---|
| Elementaranalyse | berechnet | C (60,27) | H (5,28) | N (3,06) |
| | gefunden. | C (60,03) | H (5,35) | N (3,55). |

**Beispiel 4:** 9-Fluor-fluoren-9-carbonsäurescopinester Methobromid :

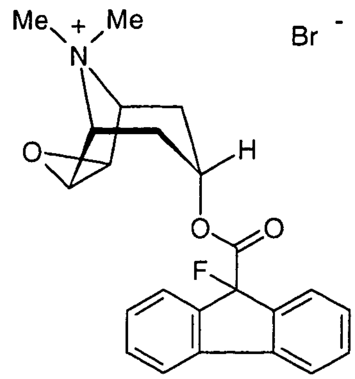

**4.1:** 9-Fluor-fluoren-9-carbonsäurescopinester **4d:**

0,885 ml (0,005 mol) Bis-(2-methoxyethyl)-aminosulfurtrifluorid werden in 25 ml Dichlormethan vorgelegt und in Analogie zur Vorgehensweise nach 2.1 mit 1,42 g (0,004 mol) **4c** umgesetzt.
Ausbeute: 1,1 g beige Kristalle (= 75 % d. Th.)

**4.2:** 9-Fluor-fluoren-9-carbonsäurescopinester -Methobromid :

1,1 g (0,003 mol) **4d** werden in 30 ml Acetonitril aufgenommen und mit 1,71 g (0,009 mol) 50%-iges Methylbromidlösung in Acetonitril in Analogie zu Schritt 1.3 umgesetzt. Zur Reinigung wird aus Isopropanol umkristallisiert.
Ausbeute: 0,45 g weiße Kristalle (= 33 % d. Th); Schmp.: 200-201°C.

| | | | | |
|---|---|---|---|---|
| Elementaranalyse | berechnet | C (60,01) | H (5,04) | N (3,04) |
| | gefunden. | C (59,91) | H (5,18) | N (3,10). |

**Beispiel 5:** 9-Methyl-fluoren-9-carbonsäuretropenolester Methobromid :

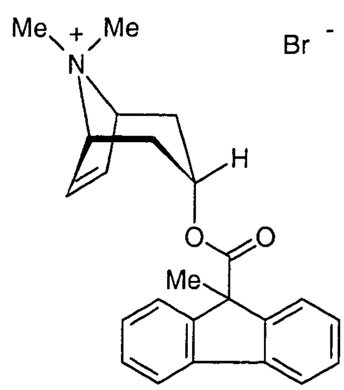

**5.1.:** 9-Methyl-fluoren-9-carbonsäure **3b:**.

a) 9-Methyl-fluoren-9-carbonsäuremethylester:

Aus 7,6 g (0,33 mol) Natrium und 300 ml Ethanol wird eine Natriumethylatlösung hergestellt, in die 69,6 g (0,33 mol) 9-Fluorencarbonsäure portionsweise zugegeben werden. Nach beendeter Zugabe wird 2,5 Stunden bei

Raumtemperatur gerührt.

Anschließend wird zur Trockene eingedampft, der Rückstand in 600 ml Dimethylformamid suspendiert und 93,96 g (0,662 mol) Methyljodid zugetropft. Es wird 3 Stunden bei konstanter Temperatur nachgerührt. Die trübe Lösung wird unter Kühlung in 500 ml Wasser und 300 ml Diethylether eingerührt und extrahiert, die organische Phase mit Wasser und 10%iger Natriumcarbonatlösung gewaschen, getrocknet und zur Trockene eingedampft. Der Rückstand wird mittels Säulenchromatographie, Laufmittel: Cyclohexan / Ethylacetat 96:4 gereinigt. Ausbeute: 12,61 g weiße Kristalle (= 16% d. Th.); Schmp.: 108°-109° C.

b) 9-Methyl-fluoren-9-carbonsäure **3b:**

12,6 g (0,053 mol) 9-Methyl-fluoren-9-carbonsäuremethylester und 53 ml 2 molare, wässerige Natriumhydroxidlösung werden in 120 ml 1,4-Dioxan 24 Stunden bei Raumtemperatur gerührt. Das Dioxan wird abdestilliert, mit Wasser auf 300 ml Gesamtvolumen versetzt und mit Diethylether extrahiert. Die wässrige Phase wird mit 3 molarer, wässeriger HCl angesäuert, kristallisiert und filtriert.
Ausbeute: 11,25 g weiße Kristalle (= 95% d. Th.); Schmp.: 168°-169° C.

**5.2:** 9-Methyl-fluoren-9-carbonsäuretropenolester **4e**:

6,73 g (0,03 mol) **3b** werden in 60 ml Dichlormethan suspendiert, mit 5,0 g Oxalylchlorid und 1 Tropfen Dimethylformamid versetzt, anschließend eine Stunde bei Raumtemperatur gerührt und abschließend das Lösemittel abdestilliert. Das verbleiebende Säurechlorid wird ohne weitere Reinigung in den nächsten Schritt eingesetzt.
4,18 g (0,03 mol) Tropenol und 4,27 g (0,033 mol) Diisopropylethylamin werden in 100 ml Dichlorethan suspendiert, das Säurechlorid in 30 ml Dichlorethan bei 35-40°C zugetropft und anschließend 24 Stunden bei 40° C gerührt. Die Suspension wird mit Dichlormethan verdünnt und verdünnter Sälzsäure extrahiert. Die organische Phase wird anschließend mit Wasser gewaschen, über $MgSO_4$ getrocknet und das Produkt mit einer Lösung von HCl in Diethylether in sein Hydrochlorid überführt. Das Löseittel wird anschließend entfernt. Zur Reinigung wird das gefällte Hydrochlorid in Wasser aufgenommen und mit Diethylether extrahiert. Die wässerige Phase wird mit 10%iger aq. Natriumcarbonatlösung basisch gestellt und mit Dichlormethan extrahiert. Die organische Phase wird über $MgSO_4$ getrocknet und das Lösemittel abdestilliert. Ausbeute: 4,40 g gelbes Öl (= 42% d. Th.);

**5.3:** 9-Methyl-fluoren-9-carbonsäuretropenolester Methobromid :

1,8 g (0,005 mol) der freien Base **4e** werden analog zur Durchführung unter Stufe 1.3 umgesetzt. Die Reinigung erfolgt durch Umkristallisation aus Aceton.
Ausbeute: 1,80 g weiße Kristalle (= 82 % d. Th.); Schmp.: 258-259°C;

| | | | | |
|---|---|---|---|---|
| Elementaranalyse | berechnet | C (65,46) | H (5,95) | N (3,18) |
| | gefunden. | C (64,15) | H (5,95) | N (3,18). |

**Beispiel 6**: 9-Methyl-fluoren-9-carbonsäurescopinester Methobromid :

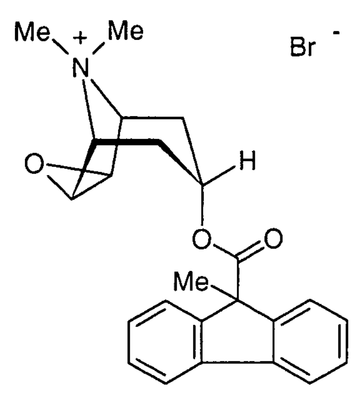

**6.1:** 9-Methyl-fluoren-9-carbonsäurescopinester **4f:**

2,5 g (0,007 mol) Tropenolester **4e** werden mit 0,13 g (0,001 mol) Vanadium-(V)-oxid und 1,43 g (0,015 mol) $H_2O_2$-Harnstoff in Analogie zur Vorgehensweise nach Stufe 3.1 umgesetzt. Ausbeute: 1,8 g weiße Kristalle (= 71 % d. Th.).

**6.2:** 9-Methyl-fluoren-9-carbonsäurescopinester -Methobromid :

1,8 g (0,005 mol) **4f** werden in 30 ml Acetonitril aufgenommen und mit 2,848 g (0,015 mol) 50%-iges Methylbromidlösung in Acetonitril in Analogie zu Schritt 1.3 umgesetzt. Ausbeute: 1,6 g weiße Kristalle (= 70 % d. Th); Schmp.: 214°C.

| | | | | |
|---|---|---|---|---|
| Elementaranalyse | berechnet | C (62,13) | H (5,93) | N (4,26) |
| | gefunden. | C (62,23) | H (6,05) | N (4,32). |

Wie gefunden wurde, stellen die erfindungsgemäßen Verbindungen der Formel **1** Antagonisten des M3- Rezeptors (Muskarinischer Rezeptorsubtyp 3) dar. Die erfindungsgemäßen Verbindungen weisen bezüglich der Affinität zum M3-Rezeptor Ki-Werte von kleiner 10nM auf. Diese Werte wurde entsprechend der nachfolgend beschriebenen Vorgehensweise bestimmt.

Chemikalien

3H-NMS wurde von der Firma Amersham, Braunschweig, mit einer spezifischen Radioaktivität von 3071 GBq/mmol (83 Ci/mmol) bezogen. Alle weiteren Reagentien wurden von Serva, Heidelberg und von Merck, Darmstadt erhalten.

Zellmembranen:

Wir setzten Zellmembranen von CHO Zellen (Chinese hamster ovary) ein, die mit den entsprechenden Genen der humanen muskarinischen Rezeptorsubtypen hm1 bis hm5 transfiziert waren (BONNER). Die Zellmembranen des gewünschten Subtypes wurden aufgetaut, resuspendiert per Hand mit einem Glas-Homogenisator und mit HEPES-Puffer auf eine Endkonzentration von 20-30 mg Protein/ml verdünnt.

Rezeptorbindungsstudien:

Der Bindungsassay wurde in einem Endvolumen von 1 ml ausgeführt und setzte sich zusammen aus 100 µl unmarkierter Substanz in verschiedenen Konzentrationen, 100 µl Radioligand (3H-N-Methylscopolamin 2 nmol/L (3H-NMS), 200 µl Membranpräparation und 600 µl HEPES Puffer (20 mmol/L HEPES, 10 mmol/L MgCl2, 100 mmol/L NaCl, mit 1 mol/L NaOH auf pH 7.4 eingestellt).
Die unspezifische Bindung ermittelten wir durch 10 µmol/L Atropin.
Die Inkubation von 45 min. erfolgte bei 37°C in 96-well Mikrotiterplatten (Beckman, Polystyrol, Nr. 267001) als Doppelbestimmung. Die Inkubation endete durch Filtration mittels Inotech Zellernter (Typ IH 110) über Whatman G-7 Filter. Die Filter wurden mit 3 ml eisgekühltem HEPES Puffer gewaschen und vor der Messung getrocknet.

Bestimmung der Radioaktivität:

Die Radioaktivität der Filtermatten wurden simultan mittels zweidimensionalem, digitalem Autoradiograph (Berthold, Wildbad, Typ 3052) gemessen.

Auswertung:

Die Ki-Werte berechneten wir mit impliziten Gleichungen, die direkt vom Massenwirkungsgesetz abgeleitet wurden, mit dem Modell für die 1 Rezeptor 2 Liganden Reaktion (SysFit - Software, SCHITTKOWSKI).

Literatur:

BONNER TI, New subtypes of muscarinic acetylcholine receptors

Trends Pharmacol. Sci. 10, Suppl.: 11-15 (1989); SCHITTKOWSKI K
Parameter estimation in systems of nonlinear equations Numer Math. 68: 129-142 (1994).

Die erfindungsgemäßen Verbindungen der Formel 1 zeichnen sich durch vielfältige Anwendungsmöglichkeiten auf therapeutischem Gebiet aus.
Hervorzuheben sind erfindungsgemäß solche Anwendungsmöglichkeiten, für welche die erfindungsgemäßen Verbindungen der Formel 1 aufgrund ihrer pharmazeutischen Wirksamkeit als Anticholinergikum bevorzugt zur Anwendung gelangen können.

Dies sind beispielsweise die Therapie von Asthma oder COPD (chronic obstructive pulmonary disease = chronisch obstruktive Lungenerkrankung). Die Verbindungen der allgemeinen Formel 1 können ferner zur Behandlung vagal bedingter Sinusbradykardien und zur Behandlung von Herz-Rhythmus-Störungen zum Einsatz gelangen. Generell lassen sich die erfindungsgemäßen Verbindungen ferner zur Behandlung von Spasmen beispielsweise im Gastrointestinaltrakt mit therapeutischem Nutzen einsetzen. Sie können ferner bei der Behandlung von Spasmen in harnableitenden Wegen sowie beispielsweise bei Menstruationsbeschwerden zum Einsatz gelangen.
Von den vorstehend beispielhaft aufgeführten Indikationsgebieten, kommt der Therapie von Asthma und COPD mittels der erfindungsgemäßen Verbindungen der Formel 1 eine besondere Bedeutung zu.

Die Verbindungen der allgemeinen Formel 1 können allein oder in Kombination mit anderen erfindungsgemäßen Wirkstoffen der Formel 1 zur Anwendung gelangen. Gegebenenfalls können die Verbindungen der allgemeinen Formel 1 auch in Kombination mit weiteren pharmakologisch aktiven Wirkstoffen eingesetzt werden. Es handelt sich hierbei insbesondere um Betamimetica, Antiallergika, PAF-Antagonisten, PDE IV-Inhibitoren, Leukotrien-Antagonisten, p38 Kinase-Inhibitoren, EGFR-Kinase-Hemmer und Corticosteroiden, sowie Wirkstoffkombinationen davon.

Als Beispiel für Betamimetika, die erfindungsgemäß mit den Verbindungen der Formel 1 als Kombination zum Einsatz kommen können, seien genannt Verbindungen, die ausgewählt sind aus der Gruppe bestehend aus Bambuterol, Bitolterol, Carbuterol, Clenbuterol, Fenoterol, Formoterol, Hexoprenalin, Ibuterol, Pirbuterol, Procaterol, Reproterol, Salmeterol, Sulfonterol, Terbutalin, Tolubuterol, 4-Hydroxy-7-[2-{[2-{[3-(2-phenylethoxy)propyl]sulfonyl}ethyl]-amino}ethyl]-2(3H)-benzothiazolon, 1-(2-Fluoro-4-hydroxyphenyl)-2-[4-(1-benzimidazolyl)-2-methyl-2-butylamino] ethanol, 1-[3-(4-Methoxybenzyl-amino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-nbutyloxyphenyl)-2-methyl-2-propylamino]ethanol, 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-Hydroxy-8-(1-hydroxy-2-isopropylaminobutyl)-2H-1,4-benzoxazin-3-(4H)-on, 1-(4-Amino-3-chloro-5-trifluormethylphenyl)-2-tert.butylamino)ethanol und 1-(4-Ethoxycarbonylamino-3-cyano-5-fluorophenyl)-2-(tert.butylamino)ethanol, gegebenenfalls in Form ihrer Racemate, ihrer Enantiomere, ihrer Diastereomere, sowie gegebenenfalls ihrer pharmakologisch unbedenklichen Säureadditionssalze und Hydrate. Besonders bevorzugt gelangen als Betamimetika solche Wirkstoffe in Kombination mit den erfindungsgemäßen Verbindungen der Formel 1 zur Anwendung, die ausgewählt sind aus der Gruppe bestehend aus Fenoterol, Formoterol, Salmeterol, 1-[3-(4-Methoxybenzyl-amino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino]ethanol,1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, gegebenenfalls in Form ihrer Racemate, ihrer Enantiomere, ihrer Diastereomere, sowie gegebenenfalls ihrer pharmakologisch unbedenklichen Säureadditionssalze, ihrer Solvate und/oder ihrer Hydrate. Von den vorstehend genannten Betamimetika kommt hierbei den Verbindungen Formoterol und Salmeterol gegebenenfalls in Form ihrer Racemate, ihrer Enantiomere, ihrer Diastereomere, sowie gegebenenfalls ihrer pharmakologisch unbedenklichen Säureadditionssalze und Hydrate besondere Bedeutung zu.
Erfindungsgemäß bevorzugt sind die Säureadditionssalze der Betamimetika beispielsweise ausgewählt aus der Gruppe bestehend aus Hydrochlorid, Hydrobromid, Sulfat, Phosphat, Fumarat, Methansulfonat und Xinafoat. Besonders bevorzugt sind die Salze im Falle des Salmeterols ausgewählt aus Hydrochlorid, Sulfat und Xinafoat, von denen das Xinafoate besonders bevorzugt ist. Besonders bevorzugt sind die Salze im Falle des Formoterols ausgewählt aus Hydrochlorid, Sulfat und Fumarat, von denen das Hydrochlorid und Fumarat besonders bevorzugt sind. Erfindungsgemäß von herausragender Bedeutung ist Formoterolfumarat.

Im Rahmen der vorliegenden Erfindung werden unter Corticosteroiden, die gegebenenfalls in Kombination mit den Verbindungen der Formel 1 zum Einsatz gelangen können, Verbindungen verstanden, die ausgewählt sind aus der Gruppe bestehend aus Flunisolide, Beclomethasone, Triamcinolone, Budesonid, Fluticasone, Mometasone, Ciclesonide, Rofleponide, GW 215864, KSR 592, ST-126 und Dexametasone. Bevorzugt sind im Rahmen der vorliegenden Erfindung die Corticosteroide ausgewählt aus der Gruppe bestehend aus Flunisolide, Beclomethasone, Triamcinolone, Budesonid, Fluticasone, Mometasone, Ciclesonide und Dexametasone, wobei hier dem Budesonid, Fluticasone, Mometasone und Ciclesonide, insbesondere dem Budesonid und dem Fluticason eine besondere Bedeutung

zukommt. Gegebenfalls wird im Rahmen der vorliegenden Patentanmeldung statt der Bezeichnung Corticosteroide auch nur die Bezeichnung Steroide verwendet. Eine Bezugnahme auf Steroide schließt im Rahmen der vorliegenden Erfindung eine Bezugnahme auf Salze oder Derivate, die von den Steroiden gebildet werden können, mit ein. Als mögliche Salze oder Derivate werden beispielsweise genannt: Natriumsalze, Sulfobenzoate, Phosphate, Isonicotinate, Acetate, Propionate, Dihydrogenphosphate, Palmitate, Pivalate oder Furoate. Gegebenenfalls können die Corticosteroide auch in Form ihrer Hydrate vorliegen.

Als Beispiel für PDE-IV-Inhibitoren, die erfindungsgemäß mit der Verbindung der Formel **1** als Kombination zum Einsatz kommen können, seien genannt Verbindungen, die ausgewählt sind aus der Gruppe bestehend aus Enprofylline, Roflumilast, Ariflo, Bay-198004, CP-325,366, BY343, D-4396 (Sch-351591), V-11294A und AWD-12-281. Bevorzugte PDE-IV-Inhibitoren sind ausgewählt aus der Gruppe bestehend aus Enprofylline, Roflumilast, Ariflo und AWD-12-281, wobei AWD-12-281 als Kombinationspartner mit der erfindungsgemäßen Verbindung der Formel **1** besonders bevorzugt ist. Eine Bezugnahme auf die vorstehend genannten PDE-IV-Inhibitoren schließt im Rahmen der vorliegenden Erfindung eine Bezugnahme auf deren gegebenenfalls existierende pharmakologisch verträgliche Säureadditionssalze ein. Unter den physiologisch verträglichen Säureadditionssalzen, die von den vorstehend genannten PDE-IV-Inhibitoren gebildet werden können, werden erfindungsgemäß pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure sind. Erfindungsgemäß bevorzugt sind in diesem Zusammenhang die Salze ausgewählt aus der Gruppe bestehend aus Acetat, Hydrochlorid, Hydrobromid, Sulfat, Phosphat und Methansulfonat.

Im Rahmen der vorliegenden Erfindung werden unter Dopamin-Agonisten, die gegebenenfalls in Kombination mit den Verbindungen der Formel **1** zum Einsatz gelangen können, Verbindungen verstanden, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan. Bevorzugt werden im Rahmen der vorliegenden Erfindung Dopamin-Agonisten als Kombinationspartner mit den Verbindungen der Formel **1** eingesetzt, die ausgewählt sind aus der Gruppe bestehend aus Pramipexol, Talipexol und Viozan, wobei Pramipexol eine besondere Bedeutung zukommt. Eine Bezugnahme auf die vorstehend genannten Dopamin-Agonisten schließt im Rahmen der vorliegenden Erfindung eine Bezugnahme auf deren gegebenenfalls existierende pharmakologisch verträgliche Säureadditionssalze und gegebenenfalls deren Hydrate ein. Unter den physiologisch verträglichen Säureadditionssalzen, die von den vorstehend genannten Dopaminagonisten gebildet werden können, werden beispielsweise pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure sind.

Als Beispiel für Antiallergika, die erfindungsgemäß mit den Verbindungen der Formel **1** als Kombination zum Einsatz kommen können, seien genannt Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin. Bevorzugte Antiallergika, die im Rahmen der vorliegenden Erfindung in Kombination mit den erfindungsgemäßen Verbindungen der Formel **1** zum Einsatz gelangen können, sind ausgewählt aus der Gruppe bestehend aus Epinastin, Cetirizin, Azelastin, Fexofenadin, Levocabastin, Loratadin, Ebastin, Desloratidin und Mizolastin wobei Epinastin und Desloratidin besonders bevorzugt sind. Eine Bezugnahme auf die vorstehend genannten Antiallergika schließt im Rahmen der vorliegenden Erfindung eine Bezugnahme auf deren gegebenenfalls existierende pharmakologisch verträgliche Säureadditionssalze ein.

Als Beispiel für PAF-Antagonisten, die erfindungsgemäß mit den Verbindungen der Formel **1** als Kombination zum Einsatz kommen können seien genannt 4-(2-Chlorphenyl)-9-methyl-2-[3(4-morpholinyl)-3-propanon-1-yl]-6Hthieno-[3,2-f][1,2,4]triazolo[4,3-a][1,4]diazepin, 6-(2-Chlorphenyl)-8,9-dihydro-1-methyl-8-[(4-morpholinyl)carbonyl]-4H,7H-cyclopenta-[4,5]thieno-[3,2-f][1,2,4]triazolo[4,3-a][1,4]diazepin.

Als Beispiel für EGFR-Kinase-Hemmer, die mit den erfindungsgemäßen Verbindungen der Formel **1** als Kombination zum Einsatz kommen können, seien als besonders bevorzugt genannt 4-[(3-Chlor-4-fluor-phenyl)amino]-7-[4-((R)-6-methyl-2-oxo-morpholin-4-yl)-butyloxy]-6-[(vinylcarbonyl)amino]-chinazolin, 4-[(3-Chlor-4-fluorphenyl)amino]-7-[4-((S)-6-methyl-2-oxo-morpholin-4-yl)-butyloxy]-6-[(vinylcarbonyl)arnino]-chinazolin, 4-[(3-Chlor-4-fluor-phenyl)amino]-7-(2-{4-[(S)-(2-oxo-tetrahydrofuran-5-yl)carbonyl]-piperazin-1-yl}-ethoxy)-6-[(vinylcarbonyl)amino]-chinazolin, 4-[(3-Chlor-4-fluor-phenyl)amino]-7-[2-((S)-6-methyl-2-oxo-morpholin-4-yl)-ethoxy]-6-[(vinylcarbonyl)amino]-chinazolin, 4-[(3-Chlor-4-fluorphenyl)amino]-6-[(4-{N-[2-(ethoxycarbonyl)-ethyl]-N-[(ethoxycarbonyl)methyl]amino}-1-oxo-2-buten-1-yl)amino]-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopropylmethoxy-chinazolin und 4-[(3-Chlor-4-fluorphenyl)amino]-6-[3-(morpholin-4-yl)-propyloxy]-7-methoxychinazolin. Eine Bezugnahme auf die vorstehend genannten EGFR-Kinase-Hemmer schließt im Rahmen der vorliegenden Erfindung eine Bezugnahme auf deren gegebenenfalls existierende pharmakologisch verträgliche Säureadditionssalze ein. Unter den physiologischbzw. pharmakologisch verträglichen Säureadditionssalzen, die von den EGFR-Kinase-Hemmern gebildet werden können, werden erfindungsgemäß pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure sind. Erfindungsgemäß bevorzugt sind die Salze der EGFR-Kinase-Hemmer ausgewählt aus den Salzen der Essigsäure, Salzsäure, Bromwasserstoffsäure, Schwefelsäure, Phosphorsäure und Methansulfonsäure.

Als Beispiel für p38 Kinase-Hemmer, die mit den erfindungsgemäßen Verbindungen der Formel **1** als Kombination zum Einsatz kommen können, seien als besonders bevorzugt genannt 1-[5-*tert*-Butyl-2-*p*-tolyl-2H-pyrazol-3-yl]-3-[4-(2-morpholin-4-ylethoxy)naphthalin-1-yl]-harnstoff; 1-[5-*tert*-Butyl-2-*p*-tolyl-2H-pyrazol-3-yl]-3-[4-(2-(1-oxothiomorpholin-4-yl)ethoxy)naphthalin-1-yl]-harnstoff;1-[5-*tert*-butyl-2-(2-methylpyridin-5-yl)-2H-pyrazol-3-yl]-3-[4-(2-pyridin-4-yl-ethoxy)naphthalin-1-yl]-harnstoff; 1-[5-*tert*-butyl-2-(2-methoxypyridin-5-yl)-2H-pyrazol-3-yl]-3-[4-(2-morpholin-4-yl-ethoxy)naphthalin-1-yl]-harnstoff oder 1-[5-*tert*-butyl-2-methyl-2H-pyrazol-3-yl]-3-[4-(2-morpholin-4-yl-ethoxy) naphthalen-1-yl]-harnstoff. Eine Bezugnahme auf die vorstehend genannten p38-Kinase-Hemmer schließt im Rahmen der vorliegenden Erfindung eine Bezugnahme auf deren gegebenenfalls existierende pharmakologisch verträgliche Säureadditionssalze ein. Unter den physiologisch bzw. pharmakologisch verträglichen Säureadditionssalzen, die von den p38-Kinasehemmern gebildet werden können, werden erfindungsgemäß pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure oder Maleinsäure sind.

Werden die Verbindungen der Formel **1** in Kombination mit anderen Wirkstoffen eingesetzt, ist von den vorstehend genannten Verbindungsklassen die Kombination mit Steroiden, PDE IV-inhibitoren oder Betamimetika besonders bevorzugt. Der Kombination mit Betamimetika, insbesondere mit langwirksamen Betamimetika kommt dabei eine besondere Bedeutung zu. Als besonders bevorzugt ist die Kombination der erfindungsgemäßen Verbindungen der Formel **1** mit Salmeterol oder Formoterol anzusehen.

Geeignete Anwendungsformen zur Applikation der Verbindungen der Formel **1** sind beispielsweise Tabletten, Kapseln, Zäpfchen, Lösungen etc.

Erfindungsgemäß von besonderer Bedeutung ist (insbesondere bei der Behandlung von Asthma oder COPD) die inhalative Applikation der erfindungsgemäßen Verbindungen. Der Anteil der pharmazeutisch wirksamen Verbindung (en) sollte jeweils im Bereich von 0,05 bis 90 Gew.-%, bevorzugt 0,1 bis 50 Gew.-% der Gesamtzusammensetzung liegen. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Lösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilflösemittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z. B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzuk-

ker) Emulgiermittel (z.B. Lignin, Sufitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Bei der erfindungsgemäß bevorzugten Applikation der Verbindungen der Formel **1** zur Therapie von Asthma oder COPD werden besonders bevorzugt inhalativ applizierbare Darreichungsformen bzw. pharmazeutische Formulierungen eingesetzt. Als inhalierbare Darreichungsformen kommen Inhalationspulver, treibgashaltige Dosieraerosole oder treibgasfreie Inhalationslösungen in Betracht. Im Rahmen der vorliegenden Erfindung sind von dem Begriff treibgasfreie Inhalationslösungen auch Konzentrate oder sterile, gebrauchsfertige Inhalationslösungen umfaßt. Die im Rahmen der vorliegenden Erfindung einsetzbaren Darreichungsformen werden im nachfolgenden Teil der Beschreibung detailliert beschrieben.

Erindungsgemäß einsetzbare Inhalationspulver können **1** entweder allein oder im Gemisch mit geeigneten physiologisch unbedenkliche Hilfsstoffen enthalten.

Sind die Wirkstoffe **1** im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zur Anwendung gelangen:

> Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.

Die Hilfsstoffe weisen im Rahmen der erfindungsgemäßen Inhalationspulver eine maximale mittlere Teilchengröße von bis zu 250μm, bevorzugt zwischen 10 und 150μm, besonders bevorzugt zwischen 15 und 80μm auf. Gegebenenfalls kann es sinnvoll erscheinen, den vorstehend genannten Hilfststoffen feinere Hilfsstofffraktionen mit einer mittleren Teilchengröße von 1 bis 9μm beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhaltionspulver mikronisierter Wirkstoff 1, vorzugsweise mit einer mittleren Teilchengröße von 0,5 bis 10μm, besonders bevorzugt von 1 bis 5μm, der Hilfsstoffmischung beigemischt. Verfahren zur Herstellung der erfindungsgemäßen Inhaltionspulver durch Mahlen und Mikronisieren sowie durch abschließendes Mischen der Bestandteile sind aus dem Stand der Technik bekannt. Die erfindungsgemäßen Inhalationspulver können mittels aus dem Stand der Technik bekannten Inhalatoren appliziert werden.

Erfindungsgemäße treibgashaltige Inhalationsaerosole können **1** im Treibgas gelöst oder in dispergierter Form enthalten. Hierbei können **1** in getrennten Darreichungsformen oder in einer gemeinsamen Darreichungsform enthalten sein, wobei **1** entweder beide gelöst, beide dispergiert oder jeweils nur eine Komponente gelöst und die andere dispergiert enthalten sein können.

Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie fluorierten Derivaten des Methans, Ethans, Propans, Butans, Cyclopropans oder Cyclobutans. Die vorstehend genannten Treibgase können dabei allein oder in Mischungen derselben zur Verwendnung kommen. Besonders bevorzugte Treibgase sind halogenierte Alkanderivate ausgewählt aus TG134a und TG227 und Mischungen derselben.

Die treibgashaltigen Inhalationsaerosole können ferner weitere Bestandteile wie Kosolventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

Die vorstehend genannten treibgashaltigen Inhalationaerosole können mittels im Stand der Technik bekannten Inhalatoren (MDIs = metered dose inhalers) appliziert werden.

Ferner kann die Applikation der erfindungsgemäßen Wirkstoffe **1** in Form von treibgasfreien Inhalationslösungen und Inhalationssuspensionen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Der relative Anteil an Ethanol gegenüber Wasser ist nicht begrenzt, bevorzugt liegt die maximale Grenze jedoch bei bis 70 Volumenprozent, insbesondere bei bis zu 60 Volumenprozent und besonders bevorzugt bei bis zu

30 Volumenprozent. Die restlichen Volumenprozente werden von Wasser aufgefüllt. Die **1** enthaltenden Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

In diesen Formulierungen kann gegebenenfalls auf den Zusatz von Editinsäure (EDTA) oder einem der bekannten Salze davon, Natriumedetat, als Stabilisator oder Komplexbildner verzichtet werden. Andere Ausführungsformen beinhalten diese Verbindung(en). In einer solchen bevorzugten Ausführungsform liegt der Gehalt bezogen auf Natriumedetat unter 100 mg / 100 ml, bevorzugt unter 50 mg/ 100ml, besonders bevorzugt unter 20 mg/ 100ml. Generell sind solche Inhalationslösungen bevorzugt, in denen der Gehalt an Natriumedetat bei 0 bis 10mg/100ml liegt.
Den treibgasfreien Inhaltionslösungen können Co-Solventien und/oder weitere Hilfsstoffe zugesetzt werden. Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester. Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien.
Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.
Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/100ml, besonders bevorzugt zwischen 5 und 20 mg/100ml enthalten.
Bevorzugte Formulierungen enthalten außer dem Lösungsmittel Wasser und dem Wirkstoff **1** nur noch Benzalkoniumchlorid und Natriumedetat.

In einer anderen bevorzugten Ausführungsform wird auf Natriumedetat verzichtet.

Die Dosierung der erfindungsgemäßen Verbindungen ist naturgemäß stark von der Applikationsart und der zu therapierenden Erkrankung abhängig. Bei inhalativer Applikation zeichnen sich die Verbindungen der Formel **1** bereits bei Dosen im µg-Bereich durch eine hohe Wirksamkeit aus. Auch oberhalb des µg-Bereichs, lassen sich die Verbindungen der Formel **1** sinnvoll einsetzen. Die Dosierung kann dann beispielsweise auch im Grammbereich liegen. Insbesondere bei nicht inhalativer Applikation können die erfindungsgemäßen Verbindungen mit höherer Dosierung appliziert werden (beispielsweise, aber nicht limitierend im Bereich von 1 bis 1000mg).

Die nachfolgenden Formulierungsbeipiele illustrieren die vorliegende Erfindung ohne sie jedoch in ihrem Umfang zu beschränken:

Pharmazeutische Formulierungsbeispiele

| A) | Tabletten | pro Tablette |
|---|---|---|
| | Wirkstoff **1** | 100 mg |

(fortgesetzt)

| A) | Tabletten | pro Tablette |
|---|---|---|
| | Milchzucker<br>Maisstärke<br>Polyvinylpyrrolidon<br>Magnesiumstearat | 140 mg<br>240 mg<br>15 mg<br>5 mg<br>500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) | Tabletten | pro Tablette |
|---|---|---|
| | Wirkstoff **1**<br>Milchzucker<br>Maisstärke<br>Mikrokristalline Cellulose<br>Polyvinylpyrrolidon<br>Natrium-carboxymethylstärke<br>Magnesiumstearat | 80 mg<br>55 mg<br>190 mg<br>35 mg<br>15 mg<br>23 mg<br>2 mg<br>400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpreßt das Gemisch zu Tabletten geeigneter Größe.

| C) | Ampullenlösung | |
|---|---|---|
| | Wirkstoff **1**<br>Natriumchlorid<br>Aqua pro inj. | 50 mg<br>50 mg<br>5 ml |

Der Wirkstoff wird bei Eigen-pH oder gegebenenfalls bei pH 5,5 bis 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

| D) | Dosieraerosol | |
|---|---|---|
| | Wirkstoff **1**<br>Sorbitantrioleat<br>Monofluortrichlormethan und<br>Difluordichlormethan 2 : 3 | 0,005<br>0,1<br><br>ad 100 |

Die Suspension wird in einen üblichen Aerosolbehälter mit Dosierventil gefüllt. Pro Betätigung werden vorzugsweise 50 µl Suspension abgegeben. Der Wirkstoff kann gewünschtenfalls auch höher dosiert werden (z.B. 0.02 Gew.-%).

| E) | Lösungen (in mg/100ml) | |
|---|---|---|
| | Wirkstoff **1**<br>Formoterolfumarat | 333.3 mg<br>333.3 mg |

(fortgesetzt)

| E) | Lösungen (in mg/100ml) | |
|---|---|---|
| | Benzalkoniumchlorid<br>EDTA<br>HCl (1n) | 10.0 mg<br>50.0 mg<br>ad pH 3.4 |

Diese Lösung kann in üblicher Art und Weise hergestellt werden.

| F) | Inhalationpulver | |
|---|---|---|
| | Wirkstoff <u>**1**</u><br>Formoterolfumarat<br>Lactose Monohydrat | 6 µg<br>6 µg<br>ad 25 mg |

Die Herstellung des Inhaltionspulvers erfolgt in üblicher Art und Weise durch Mischen der einzelnen Bestandteile.

| G) | Inhalationpulver | |
|---|---|---|
| | Wirkstoff <u>**1**</u><br>Lactose Monohydrat | 10 µg<br>ad 5 mg |

Die Herstellung des Inhaltionspulvers erfolgt in üblicher Art und Weise durch Mischen der einzelnen Bestandteile.

**Patentansprüche**

1. Verbindungen der allgemeinen Formel <u>1</u>

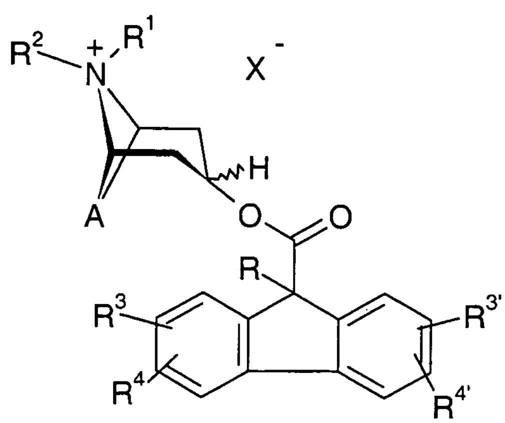

<u>**1**</u>

worin

A ein zweibindiger Rest ausgewählt aus der Gruppe bestehend aus

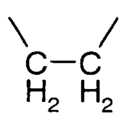 , 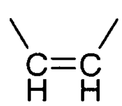 und 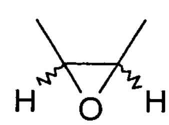 ;

$X^-$ ein einfach negativ geladenes Anion,
R Wasserstoff, Hydroxy, Methyl, Ethyl, $-CF_3$, $CHF_2$ oder Fluor;
$R^1$ und $R^2$ gleich oder verschieden, $-C_1-C_5$-Alkyl, welches gegebenenfalls durch $-C_3-C_6$-Cycloalkyl, Hydroxy oder Halogen substituiert sein kann, oder $R^1$ und $R^2$ gemeinsam eine $-C_3-C_5$-Alkylen-Brücke;
$R^3$, $R^4$, $R^{3'}$ und $R^{4'}$, gleich oder verschieden, Wasserstoff, $-C_1-C_4$-Alkyl, $-C_1-C_4$-Alkyloxy, Hydroxy, $-CF_3$, $-CHF_2$, CN, $NO_2$ oder Halogen bedeuten.

**2.** Verbindungen der Formel **1** nach Anspruch 1, worin

A ein zweibindiger Rest ausgewählt aus der Gruppe bestehend aus

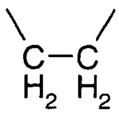 , 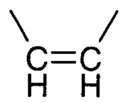 und 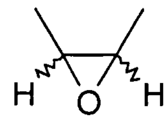 ;

$X^-$ ein einfach negativ geladenes Anion ausgewählt aus der Gruppe Chlorid, Bromid, 4-Toluolsulfonat und Methansulfonat, bevorzugt Bromid;
R Hydroxy, Methyl oder Fluor;
$R^1$ und $R^2$ gleich oder verschieden, Methyl, Ethyl oder Fluorethyl;
$R^3$, $R^4$, $R^{3'}$ und $R^{4'}$, gleich oder verschieden, Wasserstoff, Methyl, Methyloxy, Hydroxy, $-CF_3$, $-CHF_2$ oder Fluor bedeuten.

**3.** Verbindungen der allgemeinen Formel **1** nach Anspruch 1 oder 2, worin

A ein zweibindiger Rest ausgewählt aus der Gruppe bestehend aus

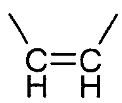 und 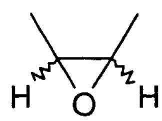 ;

$X^-$ ein einfach negativ geladenes Anion ausgewählt aus der Gruppe Chlorid, Bromid und Methansulfonat, bevorzugt Bromid;
R Hydroxy, Methyl oder Fluor, bevorzugt Methyl oder Hydroxy;
$R^1$ und $R^2$ gleich oder verschieden, Methyl oder Ethyl, bevorzugt Methyl;
$R^3$, $R^4$, $R^{3'}$ und $R^{4'}$, gleich oder verschieden, Wasserstoff, $-CF_3$, $-CHF_2$ oder Fluor, bevorzugt Wasserstoff oder Fluor bedeuten.

**4.** Verbindungen der allgemeinen Formel **1** nach Anspruch 1, 2 oder 3, worin

A ein zweibindiger Rest ausgewählt aus der Gruppe bestehend aus

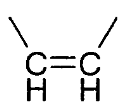 und 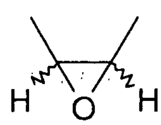 ;

$X^-$ Bromid;
R Hydroxy oder Methyl, bevorzugt Methyl;
$R^1$ und $R^2$ gleich oder verschieden, Methyl oder Ethyl, bevorzugt Methyl;
$R^3$, $R^4$, $R^{3'}$ und $R^{4'}$, gleich oder verschieden, Wasserstoff, oder Fluor bedeuten.

**5.** Verbindungen nach einem der Ansprüche 1 bis 4, gegebenenfalls in Form der einzelnen optischen Isomeren, Mischungen der einzelnen Enantiomeren oder Racemate.

**6.** Verwendung einer Verbindung der allgemeinen Formel **1** gemäß einem der Ansprüche 1 bis 5 als Arzneimittel.

**7.** Verwendung einer Verbindung der allgemeinen Formel **1** gemäß einem der Ansprüche 1 bis 5 zur Herstellung eines Arzneimittels zur Behandlung von Erkrankungen, in denen Anticholinergika einen therapeutischen Nutzen entfalten können.

**8.** Verwendung einer Verbindung der allgemeinen Formel **1** gemäß einem der Ansprüche 1 bis 5 zur Herstellung eines Arzneimittels zur Behandlung von Asthma, COPD, vagal bedingter Sinusbradykardien, Herz-Rhythmus-Störungen, Spasmen im Gastrointestinaltrakt, Spasmen in harnableitenden Wegen und Menstruationsbeschwerden.

**9.** Pharmazeutische Zubereitungen, enthaltend als Wirkstoff eine oder mehrere Verbindungen der allgemeinen Formel **1** gemäß einem der Ansprüche 1 bis 5 oder deren physiologisch verträgliche Salze gegebenenfalls in Kombination mit üblichen Hilfs- und/oder Trägerstoffen.

**10.** Pharmazeutische Zubereitungen nach Anspruch 9, **dadurch gekennzeichnet daß** diese neben einer oder mehrerer der Verbindungen der Formel **1** ferner wenigstens einen weiteren Wirkstoff enthalten, der ausgewählt ist aus der Gruppe der Betamimetica, Antiallergika, PAF-Antagonisten, PDE IV-Inhibitoren, Leukotrien-Antagonisten, p38 Kinase-Inhibitoren, EGFR-Kinase-Hemmern und Corticosteroiden.

**11.** Zwischenprodukte der allgemeinen Formel **4**

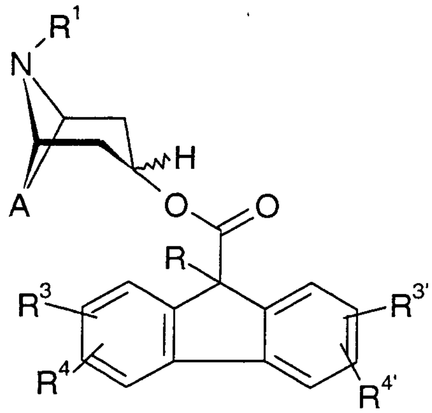

**4**

worin die Reste A, R, $R^1$, $R^3$, $R^{3'}$, $R^4$und $R^{4'}$ die in den Ansprüchen 1 bis 4 genannten Bedeutungen haben können, gegebenenfalls in Form ihrer Säureadditionssalze.

**12.** Verwendung von Verbindungen der allgemeinen Formel **2**

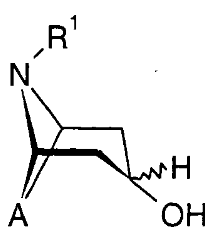

**4**

worin die Reste A und $R^1$ die in den Ansprüchen 1 bis 4 genannten Bedeutungen haben können, gegebenenfalls in Form ihrer Säureadditionssalze, zur Herstellung von Verbindungen der Formel **1** gemäß einem der Ansprüche 1 bis 5.

Europäisches Patentamt

# EUROPÄISCHER RECHERCHENBERICHT

Nummer der Anmeldung
EP 05 00 4525

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| Y,D | WO 92/16528 A (BOEHRINGER INGELHEIM INT ;BOEHRINGER INGELHEIM KG (DE)) 1. Oktober 1992 (1992-10-01) * Beispiele * | 1-12 | C07D451/12 A61K31/439 A61P1/06 A61P11/06 A61P13/06 |
| Y | BOWDEN K ET AL: "STRUCTURE-ACTIVITY RELATIONS. I. A SERIES OF ANTAGONISTS OF ACETYLCHOLINE AND HISTAMINE AT THE POSTGANGLIONIC RECEPTORS" JOURNAL OF MEDICINAL CHEMISTRY, AMERICAN CHEMICAL SOCIETY. WASHINGTON, US, 1970, Seiten 225-230, XP000960623 ISSN: 0022-2623 * Beispiele 24,25; Tabelle I * | 1-12 | |
| Y | STUBBINS J F ET AL: "MUSCARINIC M1 AND M2 BINDING AFFINITY OF CYCLIC AMINE DERIVATIVES OF CARAMIPHEN AND DICYCLOMINE" MEDICINAL CHEMISTRY RESEARCH, BIRKHAEUSER, BOSTON, US, Bd. 2, Nr. 6, 1992, Seiten 384-393, XP009011390 ISSN: 1054-2523 * Tabelle I * | 1-12 | RECHERCHIERTE SACHGEBIETE (Int.Cl.7) |
| Y | ALBANUS L: "CENTRAL AND PERIPHERAL EFFECTS OF ANTICHLINERGIC COMPOUNDS" ACTA PHARMACOLOGICA ET TOXICOLOGICA, MUNKSGAARD, DK, Bd. 28, Nr. 4, 1970, Seiten 305-326, XP009011388 ISSN: 0001-6683 * Seite 307 * | 1-12 | C07D A61K A61P |

-/--

Der vorliegende Recherchenbericht wurde für alle Patentansprüche erstellt

| Recherchenort | Abschlußdatum der Recherche | Prüfer |
|---|---|---|
| München | 14. Juni 2005 | Fazzi, R |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

1

EPO FORM 1503 03.82 (P04C03)

Europäisches Patentamt

# EUROPÄISCHER RECHERCHENBERICHT

Nummer der Anmeldung
EP 05 00 4525

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| Y | FARQUHARSON M E ET AL: "ANTAGONISM OF THE EFFECTS OF TREMORINE BY TROPINE DERIVATIVES"<br>BRITISH JOURNAL OF PHARMACOLOGY AND CHEMOTHERAPY, BRITISH MEDICAL ASSOCIATION, LONDON, GB,<br>Bd. 14, 1959, Seiten 559-566, XP009011410<br>ISSN: 0366-0826<br>* Seite 562 - Seite 563 *<br>----- | 1-12 | |
| Y | MEYERHOEFFER A ET AL: "ACID-BASE PROPERTIES OF ATROPINE, SCOPOLAMINE AND SOME GLYCOLIC ACID ESTERS"<br>ACTA CHEMICA SCANDINAVICA, MUNKSGAARD, COPENHAGEN, DK,<br>Bd. 27, Nr. 3, 1973, Seiten 868-874, XP009011391<br>ISSN: 0904-213X<br>* Seite 869 *<br>----- | 1-12 | |
| | | | RECHERCHIERTE SACHGEBIETE (Int.Cl.7) |

Der vorliegende Recherchenbericht wurde für alle Patentansprüche erstellt

| Recherchenort | Abschlußdatum der Recherche | Prüfer |
|---|---|---|
| München | 14. Juni 2005 | Fazzi, R |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

1

EPO FORM 1503 03.82 (P04C03)

**ANHANG ZUM EUROPÄISCHEN RECHERCHENBERICHT
ÜBER DIE EUROPÄISCHE PATENTANMELDUNG NR.** EP 05 00 4525

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-06-2005

| Im Recherchenbericht angeführtes Patentdokument | Datum der Veröffentlichung | Mitglied(er) der Patentfamilie | Datum der Veröffentlichung |
|---|---|---|---|
| WO 9216528 A | 01-10-1992 | DE 4108393 A1 | 17-09-1992 |
| | | AT 202778 T | 15-07-2001 |
| | | AU 662128 B2 | 24-08-1995 |
| | | AU 1345792 A | 21-10-1992 |
| | | CA 2105575 A1 | 16-09-1992 |
| | | CZ 9301917 A3 | 18-05-1994 |
| | | DE 59209907 D1 | 09-08-2001 |
| | | DK 579615 T3 | 17-09-2001 |
| | | WO 9216528 A1 | 01-10-1992 |
| | | EP 0579615 A1 | 26-01-1994 |
| | | ES 2160577 T3 | 16-11-2001 |
| | | FI 934000 A | 13-09-1993 |
| | | GR 3036792 T3 | 31-01-2002 |
| | | HU 65132 A2 | 28-04-1994 |
| | | IE 920815 A1 | 23-09-1992 |
| | | IL 101225 A | 14-05-1996 |
| | | JP 3352684 B2 | 03-12-2002 |
| | | JP 6505718 T | 30-06-1994 |
| | | MX 9201139 A1 | 01-10-1992 |
| | | NO 933274 A | 12-11-1993 |
| | | NZ 241961 A | 26-07-1995 |
| | | PL 179673 B1 | 31-10-2000 |
| | | PT 100234 A ,B | 30-07-1993 |
| | | SG 43037 A1 | 17-10-1997 |
| | | SK 94993 A3 | 06-04-1994 |
| | | US 5654314 A | 05-08-1997 |
| | | ZA 9201875 A | 13-09-1993 |

EPO FORM P0461

Für nähere Einzelheiten zu diesem Anhang : siehe Amtsblatt des Europäischen Patentamts, Nr.12/82